# EUROPEAN PATENT SPECIFICATION

(11) **EP 0 453 597 B1**
(45) Date of publication and mention of the grant of the patent: **19.04.1995**
(21) Application number: 90107834.5
(22) Date of filing: 25.04.1990
(51) Int. Cl.: C07H 19/20

(54) **Process for producing a mixture of inosinic acid and guanosinic acid by direct phosphorylation**
Verfahren zur Herstellung einer Mischung von Inosinsäure und Guanosinsäure durch direkte Phosphorylation
Procédé pour la production d'un mélange d'acide inosinique et acide guanosinique par phosphorylation directe

(43) Date of publication of application: 30.10.1991
(73) Proprietor: AJINOMOTO CO., INC., Tokyo 104 (JP)
(72) Inventor: Abe, Shigemitsu, c/o Central Research Laboratories, Kawasaki-shi, Kanagawa-ken (JP); Tsujita, Hiroshi, c/o Central Research Lab., Kawasaki-shi, Kanagawa-ken (JP); Kawakita, Tetsuya, c/o Central Research Lab., Kawasaki-shi, Kanagawa-ken (JP)
(74) Representative: Strehl Schübel-Hopf Groening & Partner

(56) References cited:
- PATENT ABSTRACTS OF JAPAN, vol. 9, no. 17 (C-262)[1740], 24th January 1985;& JP-A-59 167 599 (TAKEDA YAKUHIN KOGYO K.K.) 21-09-1984
- CHEMICAL ABSTRACTS, vol. 84, no. 3, 19th January 1976, page 504, abstract no.17681e, Columbus, Ohio, US; & SU-A-487 887 (ALL-UNION SCIENTIFIC-RESEARCH INSTITUTE OF CHEMICAL REAGENTS AND
- PURE CHEMICAL SUBSTANCES, RIGA) 15-10-1975
- CHEMICAL ABSTRACTS, vol. 75, no. 17, 25th October 1971, page 239, abstract no.118522y, Columbus, Ohio, US; Y. SANNO et al.: "Direct phosphorylation ofnucleosides with phosphorus oxychloride", & TAKEDA KENKYUSHO HO 1971,30(2), 217-24

## Description

The present invention relates to a novel method for phosphorylation of inosine and guanosine and more particularly, to a direct method for phosphorylation of the hydroxy group at the 5'-position in a simple and advantageous manner, without protecting the hydroxy groups at the 2'-position and 3'-position of the ribose moiety in the nucleoside.

The purpose is to provide an industrial and economical method for preparing a 5'-nucleotide mixture, especially a mixture of inosinic acid (inosine 5'-phosphate) and guanylic acid (guanosine-5'-phosphate), which is useful as seasoning, medical drug, etc.

For the chemical phosphorylation of unprotected nucleosides, several methods are hitherto known.

These methods can be classified as follows:
1) A method using a trialkyl phosphate as a reaction solvent:
   Tetrahedron Lett., **50**, 5065 (1967);
   Bull Chem. Soc. Jpn., **42**, 3505 (1969);
   Japanese Patent Publication No. 42-01107
2) Method using a polar organic solvent and an organic amine or its inorganic acid salt:
   Bull. Chem. Soc. Jpn., **48**, 2084 (1975)
3) Method using a tri(alkoxyalkyl) phosphate as a reaction solvent:
   Japanese Patent Application Laid-Open No. 51-86482
4) Method using a nucleoside alkali metal salt:
   USSR Patent No. 487887
5) Method using mixed crystals of guanosine and inosine:
   Japanese Patent Application Laid-Open No. 59-167599

In the past the methods of chemical phosphorylation of a nucleoside mixture, especially a mixture of inosine and guanosine, have met several problems. In general, side products are often produced in the process of forming inosinic acid and guanylic acid by phosphorylation of a mixture of free inosine and free guanosine. In particular, when free inosine is not present in an excess over free guanosine, free inosine is partially converted to by-products such as inosinic acid diphosphate, inosinic acid triphosphate, etc. via inosinic acid, by the time when the reaction of free guanosine having a slower rate of phosphorylation than inosine, is completed. In more detail, free inosine is converted into inosine-5'-monophosphate about 3 times faster than free guanosine. At the time when free guanosine is converted to more than 90% of guanosine-5'-monophosphate, free inosine is phosphorylated at positions other than the 5'-position to form many by-products such as inosine-3',5'-diphosphate, inosine-2',5'-diphosphate, inosine-5'-diphosphate, etc. For this reason, phosphorylation of a mixture of free inosine and free guanosine, especially in a mixing molar ratio of 1 : 1 is extremely difficult.

On the other hand, known methods fop the chemical phosphorylation of guanosine alone comprise adding picoline, pyridine base or its inorganic acid salts (method 2 described above) and a method using guanosine alkali metal salts (method 4 described above), in order to improve the reactivity of guanosine. According to the above described method 2, however, the yield of guanylic acid is not so improved as expected. In addition, the product is contaminated with amines which are considered to be toxic, hence the method is not suited for the application to food additives, etc.

Furthermore, in above method 4, the phosphorylation of guanosine alkali metal salts proceeds with a reaction rate twice or more as fast as the phosphorylation of guanosine. Moreover, it is known that a reactive polar solvent (a trialkyl phosphate, etc.) and a phosphorylating agent (phosphorus oxychloride, etc.) are reduced to below 1/2, respectively.

Furthermore, as an example for the phosphorylation of a mixture of inosine and guanosine, there is known a method for treating the mixture in the form of mixed crystals (method 5 described above). It is said that according to this method, the formation of by-products is minimized and the desired mixture can be obtained in a high yield. However, the method requires a step for preparing mixed crystals so that (1) the number of steps increases, (2) it is not easy to set free inosine and free guanosine in an optional mixing ratio, (3) the reaction time is considerably extended and considerable quantities of a reaction solvent and a phosphorylating agent are required.

Considering this state of the art, the chemical method for the phosphorylation of a mixture containing inosine and guanosine in an optional proportion, still involves many problems. It has therefore been desired to develop a method which allows the phosphorylation in a high yield and for a shortened reaction period using reduced amounts of reactants and with a minimized formation of side-products.

It is the task of the present invention to provide a method for the phosphorylation of a mixture of inosine and guanosine containing at least one of the alkali metal salts of inosine and guanosine, which results in a high purity and high yield of the product, by which the amount of reaction solvent and phosphorylating agent may be reduced and which may be carried out in a shortened time period.

The present inventors have carried out studies on the chemical method for mixed phosphorylation of inosine and guanosine, in which alkali metal salts are used at the closest site to the reaction site of these nucleosides to impart basicity thereto and to accelerate the phosphorylation by the anchimeric effect. By this means the reactivity of guanosine and inosine is enhanced and thus a method for preparing a mixture of inosinic acid and guanylic acid in such a high puritiy and high yield that could not be achieved heretofore, has been accomplished.

That is, the present invention relates to:
(A) A chemical process for producing a mixture of inosinic acid and guanosinic acid by mixed phosphorylation of inosine and guanosine, which is characterized by reacting inosine and guanosine containing at least one of the respective alkali metal salts of inosine and guanosine with a phosphorus oxyhalide or its hydrate in a polar organic solvent, wherein inosine and guanosine are preferably present as a guanosine alkali metal salt and free inosine and the molar ratio of said (free inosine)/(guanosine alkali metal salt) is chosen from the range of from 1/2 to 5/3;
(B) A method for mixed phosphorylation of inosine and guanosine which comprises subjecting a guanosine alkali metal salt or an inosine alkali metal salt to a phosphorylation reaction step and then subjecting the nucleoside alkali metal salt, which had not been subjected to said phosphorylation reaction step, to a phosphorylation reaction step while the transmittance of the reaction solution is in the range of 20 to 95%, in the presence of said nucleoside alkali metal salt; and,
(C) A method for mixed phosphorylation of inosine and guanosine which comprises subjecting a guanosine alkali metal salt (or free inosine) to a phosphorylation reaction step and then subjecting free inosine (or a guanosine alkali metal salt) to a phosphorylation reaction step, while the transmittance of the reaction solution is in the range of 20 to 95%, in the presence of said free inosine (or a guanosine alkali metal salt).

Hereafter, the present invention is specifically described in more detail.

The chemical mixed phosphorylation of inosine and guanosine is carried out in a reaction system in which at least one of the alkali metal salts of inosine and guanosine is present. It is preferred to use, for example, a combination of a guanosine alkali metal salt and free inosine, a combination of a guanosine alkali metal salt and an inosine alkali metal salt, free guanosine and an inosine alkali metal salt, a guanosine alkali metal salt and free inosine as well as an inosine alkali metal salt, etc. It is sufficient when the reaction system contains at least one of the alkali metal salts of guanosine and inosine. A combination containing a guanosine alkali metal salt is preferred.

By reacting the nucleosides in such a combination with the phosphorus oxyhalide or its hydrate, the mixed phosphorylation of inosine and guanosine can be carried out advantageously.

Further in the mixed phosphorylation of inosine and guanosine containing at least one of the alkali metal salts of inosine and guanosine, the phosphorylation of the nucleosides can be in various orders. Herein the order of phosphorylation is determined by the time at which the respective phosphorylations of inosine and guanosine start.

As typical examples, there is the case that the nucleosides are subjected to phosphorylation in the co-presence of the respective nucleosides from the beginning of the reaction and the case where at first one nucleoside is subjected to phosphorylation and after a first reaction period, another nucleoside is added and subjected to phosphorylation.

In the case in which the mixed phosphorylation is carried out in the co-presence of the respective nucleosides, namely, guanosine or guanosine alkali metal salt and inosine or inosine alkali metal salt are subjected to mixed phosphorylation, it is preferred that the guanosine alkali metal salt and free inosine are co-present and that the molar ratio of free inosine to the guanosine alkali metal salt is 1/2 to 5/3. In this case, recovery rates of inosinic acid and guanylic acid are more than 90%. On the other hand, in the case of a phosphorylation in which nucleosides are simultaneously added in combination of nucleosides other than those described above, the molar ratio of the inosine alkali metal salt to guanosine (or its alkali metal salt) is considerably limited to more than 9 or less than 1/9, in order to obtain inosinic acid and guanylic acid in recovery rates of more than 90%.

Next, in the case where one nucleoside is subjected to phosphorylation and another nucleoside is then added and subjected to phosphorylation, the guanosine alkali metal salt is preferably reacted in combination with the inosine alkali metal salt (or free inosine).

Specifically, after the guanosine alkali metal salt or inosine alkali metal salt is subjected to phosphorylation, the nucleoside alkali metal salt which has not been subjected to the phosphorylation is then added and subjected to phosphorylation at a transmittance (wavelength at 600 nm) of 20 to 95% in the reaction solution.

It is also possible that after the guanosine alkali metal salt is subjected to phosphorylation, free inosine is then added and subjected to phosphorylation at a transmittance (wavelength at 600 nm) of 20 to 95% in the reaction solution.

Furthermore, it is possible that after free inosine is subjected to phosphorylation, the guanosine alkali metal salt is then added and subjected to phosphorylation at a transmittance (wavelength at 600 nm) of 20 to 95% in the reaction solution.

In these cases, the molar ratio of free inosine (or its alkali metal salt) to the guanosine alkali metal salt can be freely chosen. Moreover, it is possible to ensure more than 90% of recovery rates of inosinic acid and guanylic acid. In the case of phosphorylation in combination of free guanosine and the inosine alkali metal salt described above, it is impossible to deliberately choose the molar ratio of the inosine alkali metal salt to free guanosine, in order to obtain more than 9% of recovery rates of inosinic acid and guanylic acid, but the molar ratio should be more than 9.

According to this invention, the metals for constituting the alkali metal salts of nucleosides may be any of lithium, sodium, potassium, and the like.

It is also characteristic that the nucleoside alkali metal salts used in the reaction are not restricted regarding their grain size and shape of crystals. The nucleoside alkali metal salts can be provided for the reaction simply by dispersing them in a reaction solvent as they are.

Furthermore, the water content of the nucleoside alkali metal salt should be 2% or less. It is desired to set the water content to less than 1%. This is because as the water content increases, the amount of phosphorus oxychloride must be increased so that amounts of phosphates or sodium chloride formed by neutralization after the phosphorylation increase and these substances greatly affect the purification of sodium inosinate and sodium guanylate.

The reaction solvent used may be any polar organic solvent and there may be used tri-lower alkyl phosphates, for example, triethyl phosphate, trimethyl phosphate, etc.; tri(alkoxyalkyl) phosphates such as tri(ethoxyethyl) phosphate, tri(methoxyethyl) phosphate, etc., ethylene glycol dialkyl ethers such as ethylene glycol dimethyl ether, ethylene glycol diethyl ether, etc.; cyclic ethers such as tetrahydrofuran, 1,4-dioxan, etc.; halogenated hydrocarbons such as dichloromethane, chloroform, 1,2-dichloroethane, etc.; nitro compounds such as nitromethane, nitroethane, nitrobenzene, etc. These solvents are used in the 5- to 50-fold molar amount preferably 10- to 20-fold molar amount, based on the mols of the nucleoside.

As the phosphorylating agent, a phosphorus oxyhalide or hydrates thereof can be used. Examples include phosphorus oxychloride, phosphorus oxybromide, etc. The amount of the phosphorylating agent may be 1 to 5 moles based on one mole of the nucleoside.

The reaction temperature may be desirably in the range of from -30° to 50°C since the addition of the phosphorylating agent generates heat and the temperature rapidly increases to some extent at the beginning of the reaction. By preventing the temperature elevation, the formation of diphosphates and similar by-products can be inhibited.

In the mixed phosphorylation as described above, the reaction time is approximately 30 minutes to 10 hours. Compared to the case of using the nucleoside alone, the reaction time can be shortened to about 1/2 to 1/4. In addition, the amounts of the reaction solvent and the phosphorylating agent can be reduced to about 1/2, compared to the case of using the nucleosides alone.

After completion of the phosphorylation, the reaction solution is added to more than the 20-fold molar amount of ice-cooled water to hyrolyze the phosphorylating agent. Further by adding an alkali (sodium hydroxide, etc.) thereto, the pH is adjusted to 6 to 10 to produce a solution of the alkali metal salts of inosinic acid and guanylic acid. The resulting mixture of these nucleotides can be purified by various methods. For example, the reaction solvent may be extracted with an organic solvent; or where a solvent having a lower boiling point is used, the mixture is adjusted to a pH value at which the nucleotides are stable, for example, in the case of inosinic acid, adjusted pH to about 10, followed by distillation under reduced pressure. By removing the organic solvent from the nucleotide solution obtained by these methods using activated charcoal, etc., crystals can be readily obtained.

According to the present invention, the amounts of by-products such as inosine diphosphates, etc. can be reduced and the mixture of inosinic acid and guanylic acid can be obtained in a high yield. In addition, the reaction time can be shortened and amounts of raw materials can be reduced.

In the attached drawings

Fig. 1 shows the transmittance in phosphorylation of sodium guanosine with passage of time.

Fig. 2 shows the reaction yield of inosinic acid at each transmittance in phosphorylation of sodium guanosine, when sodium inosine is added.

Hereafter the present invention is specifically described by referring to the examples and comparative examples.

### Example 1

### (Relationship between the addition time of sodium inosine and the reaction yield)

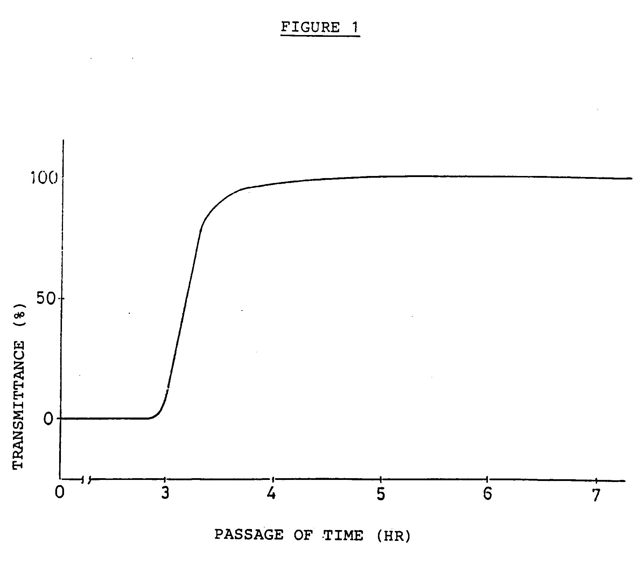

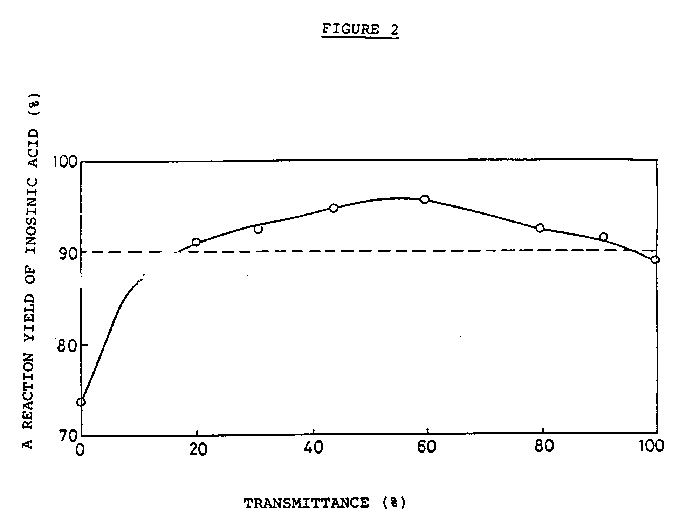

In 140 g (770 mmols) of triethyl phosphate was dispersed 10 g (33 mmols) of sodium guanosine. The dispersion was kept at -5°C and 18 g (118 mmols) of phosphorus oxychloride was added to the dispersion to initiate the reaction. The intensity of transmitted light (wavelength at 600 nm) of the reaction solution was determined by optical fiber type processing densitometer Model K395 (manufactured by Anritsu Electric Co., Ltd.). At each transmittance shown in Fig. 1, 9.5 g (33 mmols) of sodium inosine was added to perform the reaction for 6 hours, whereafter the reaction yield of sodium inosine was determined. The results are shown in Fig. 2. The reaction yield of sodium guanosine was 93 to 96% in any case. At this point, sodium inosine showed a reaction yield of 90%, when the addition time was at a transmittance between 20% and 95%.

### Example 2

In 140 g (770 mmols) of triethyl phosphate was dispersed 10 g (33 mmols) of sodium guanosine. The dispersion was cooled to -10°C and 18 g (118 mmols) of phosphorus oxychloride was added to the dispersion and the mixture was reacted for 3 hours. To the reaction mixture was added 9.5 g (33 mmols) of sodium inosine followed by reacting for 2 hours. Thereafter the reaction mixture was poured into chilled water to hydrolyze phosphorus oxychloride. Reaction yields of guanylic acid and inosinic acid were 96.6% and 97.2%, respectively.

### Example 3

In 490 g (2.7 mmols) of triethyl phosphate was dispersed 10 g (37 mmols) of inosine. The dispersion was cooled to -10°C and 61 g (400 mmols) of phosphorus oxychloride was added to the dispersion and the mixture was reacted. One hour after, 57 g (187 mmols) of sodium guanosine was added to the reaction mixture followed by reacting for 5 hours. Thereafter the reaction mixture was hydrolyzed with water cooled to 5°C. Reaction yields of inosinic acid and guanylic acid were 97.5% and 95.8%, respectively.

### Example 4

In 156 g (864 mmols) of triethyl phosphate were dispersed 10 g (37 mmols) of inosine and 11 g (36 mmols) of sodium guanosine. The disperion was cooled to -10°C and 20 g (130 mmols) of phosphorus oxychloride was added to the dispersion and the mixture was reacted for 5 hours. Thereafter, the reaction solution was poured into water cooled to 5°C to hydrolyze phosphorus oxychloride. At this point, reaction yields of inosinic acid and guanylic acid were 95.2% and 96.8%, respectively.

### Example 5

In 446 g (2.5 mmols) of triethyl phosphate were dispersed 50 g (164 mmols) of sodium guanosine and 24 g (83 mmols) of inosine. The dispersion was cooled to -10°C and 75 g (494 mmols) of phosphorus oxychloride was added to the dispersion and the mixture was reacted for 5 hours. Thereafter, the reaction solution was poured into water cooled to 5°C to hydrolyze phosphorus oxychloride. At this point, reaction yields of guanylic acid and inosinic acid were 96.5% and 97.0%, respectively.

### Comparative Example 1

Guanosine, 50 g (164 mmols), was mixed with 48 g (164 mmols) of inosine and the mixture was dispersed in 550 g of triethyl phosphate. The solution was cooled to -10°C and 115 g (754 mmols) of phosphorus oxychloride was added to the dispersion and the mixture was reacted for 6 hours. The reaction solution was hydrolyzed with water at 5°C and yields of guanylic acid and inosinic acid thus obtained were 85% and 94%, respectively.

### Comparative Example 2

Guanosine, 50 g (164 mmols), was mixed with 48 g (164 mmols) of inosine and the mixture was dispersed in 350 g of acetonitrile. To the solution was added 137 g of pyridine and the mixture was cooled to -10°C. After 120 g (787 mmols) of phosphorus oxychloride had been added, the mixture was reacted for 6 hours. Phosphorus oxychloride in the reaction solution was hydrolyzed with water at 5°C to give guanylic acid and inosinic acid. In this case, yields of guanylic acid and inosinic acid thus obtained were 85.1% and 93.2%, respectively.

### Comparative Example 3

Guanosine, 50 g (164 mmols), was mixed with 9.6 g (32 mmols) of inosine and the mixture was dispersed in 780 g (4.3 mols) of triethyl phosphate. The dispersion was cooled to -10°C and 69 g (450 mmols) of phosphorus oxychloride was added to the dispersion. The mixture was reacted for 10 hours. The reaction solution was hydrolzyed with water at 5°C and the yields of guanylic acid and inosinic acid thus obtained were 85% and 88%, respectively.

## Claims

1. A process for producing a mixture of inosinic acid and guanosinic acid by direct phosphorylation of the 5'-hydroxy group of inosine and guanosine, characterized in that a mixture of inosine and guanosine containing at least one alkali metal salt selected from alkali metal salts of inosine and/or guanosine, is reacted with a phosphorus oxyhalide or its hydrate in a polar organic solvent.

2. A process according to claim 1, wherein said inosine and guanosine are present in the reaction mixture in the form of a guanosine alkali metal salt and free inosine, wherein the molar ratio of said free inosine/guanosine alkali metal salt is in the range of from 1/2 to 5/3.

3. A process for producing a mixture of inosinic acid and guanosinic acid by direct phosphorylation of the 5'-hydroxy group of inosine and guanosine, characterized in that a first nucleoside alkali metal salt, selected from a guanosine alkali metal salt or an inosine alkali metal salt is subjected to a phosphorylation reaction step and thereafter a second nucleoside alkali metal salt which had not yet been phosphorylated is subjected to a phosphorylation reaction step in the presence of said first nucleoside alkali metal salt, while the transmittance of the reaction solution is in the range of 20 to 95%.

4. A process for producing a mixture of inosinic acid and guanosinic acid by direct phosphorylation of the 5'-hydroxy group of inosine and guanosine, which comprises subjecting a guanosine alkali metal salt to a phosphorylation reaction step and then subjecting free inosine to a phosphorylation reaction step in the presence of said guanosine alkali metal salt while the transmittance of the reaction solution is in the range of 20 to 95%.

5. A process for producing a mixture of inosinic acid and guanosinic acid by direct phosphorylation of the 5'-hydroxy group of inosine and guanosine, which comprises subjecting free inosine to a phosphorylation reaction step and then subjecting a guanosine alkali metal salt to a phosphorylation reaction step in the presence of said free inosine while the transmittance of the reaction solution is in the range of 20 to 95%.

## Patentansprüche

1. Verfahren zur Herstellung eines Gemisches aus Inosinsäure und Guanosinsäure durch direkte Phosphorylierung der 5'-Hydroxygruppe von Inosin und Guanosin, dadurch gekennzeichnet, daß ein Gemisch aus Inosin und Guanosin, das mindestens ein unter Alkalimetallsalzen des Inosins und/oder Guanosins ausgewähltes Alkalimetallsalz enthält, mit einem Phosphoroxyhalogenid oder dessen Hydrat in einem polaren organischen Lösungsmittel umgesetzt wird.

2. Verfahren gemäß Anspruch 1, worin das Inosin und Guanosin im Reaktionsgemisch in Form eines Alkalimetallsalzes des Guanosins und freiem Inosin vorhanden sind, wobei das Molverhältnis freies Inosin/Alkalimetallsalz des Guanosins im Bereich von 1/2 bis 5/3 liegt.

3. Verfahren zur Herstellung eines Gemisches aus Inosinsäure und Guanosinsäure durch direkte Phosphorylierung der 5'-Hydroxygruppe von Inosin und Guanosin, dadurch gekennzeichnet, daß ein erstes, unter einem Alkalimetallsalz des Guanosins oder einem Alkalimetallsalz des Inosins ausgewähltes Nucleosid-Alkalimetallsalz einer Phosphorylierungsstufe unterzogen wird, und anschließend ein zweites, bislang noch nicht phosphoryliertes Nucleosid-Alkalimetallsalz in Gegenwart des ersten Nucleosid-Alkalimetallsalzes einer Phosphorylierungsstufe unterzogen wird, wobei sich die Durchlässigkeit der Reaktionslösung in einem Bereich von 20 bis 95 % befindet.

4. Verfahren zur Herstellung eines Gemisches aus Inosinsäure und Guanosinsäure durch direkte Phosphorylierung der 5'-Hydroxygruppe von Inosin und Guanosin, welches das Unterziehen eines Alkalimetallsalzes des Guanosins einer Phosphorylierungsstufe und anschließendes Unterziehen von freiem Inosin einer Phosphorylierungsstufe in Gegenwart des Alkalimetallsalzes des Guanosins umfaßt, wobei sich die Durchlässigkeit der Reaktionslösung im Bereich von 20 bis 95 % befindet.

5. Verfahren zur Herstellung eines Gemisches aus Inosinsäure und Guanosinsäure durch direkte Phosphorylierung der 5'-Hydroxygruppe des Inosins und Guanosins, welches das Unterziehen von freiem Inosin einer Phosphorylierungsstufe und anschließendes Unterziehen eines Alkalimetallsalzes des Guanosins einer Phosphorylierungsstufe in Gegenwart des freien Inosins umfaßt, wobei sich die Durchlässigkeit der Reaktionslösung im Bereich von 20 bis 95 % befindet.

## Revendications

1. Procédé de préparation d'un mélange d'acide inosinique et d'acide guanosinique par phosphorylation directe du groupe 5'-hydroxy d'inosine et de guanosine, caractérisé en ce qu'un mélange d'inosine et de guanosine contenant au moins un sel de métal alcalin choisi parmi des sels de métal alcalin d'inosine et/ou de guanosine est mis à réagir avec un oxyhalogénure de phosphore ou son hydrate dans un solvant organique polaire.

2. Procédé suivant la revendication 1, caractérisé en ce que l'inosine et la guanosine sont présentes dans le mélange réactionnel sous la forme d'un sel de métal alcalin de guanosine et d'inosine libre, le rapport molaire inosine libre/sel de métal alcalin de guanosine étant de l'ordre de 1/2 à 5/3.

3. Procédé de préparation d'un mélange d'acide inosinique et d'acide guanosinique par phosphorylation directe du groupe 5'-hydroxy d'inosine et de guanosine, caractérisé en ce qu'un premier sel de métal alcalin de nucléoside, choisi parmi un sel de métal alcalin de guanosine et un sel de métal alcalin d'inosine, est soumis à une étape réactionnelle de phosphorylation et en ce qu'ensuite un deuxième sel de métal alcalin de nucléoside, qui n'a pas encore été phosphorylé, est soumis à une étape réactionnelle de phosphorylation en présence du premier sel de métal alcalin de nucléoside, tandis que la transmission de la solution réactionnelle est de l'ordre de 20 à 95 %.

4. Procédé de préparation d'un mélange d'acide inosinique et d'acide guanosinique par phosphorylation directe du groupe 5'-hydroxy d'inosine et de guanosine, comprenant une exposition d'un sel de métal alcalin de guanosine à une étape réactionnelle de phosphorylation et ensuite une exposition d'inosine libre à une étape réactionnelle de phosphorylation en présence du sel de métal alcalin de guanosine, tandis que la transmission de la solution réactionnelle est de l'ordre de 20 à 95 %.

5. Procédé de préparation d'un mélange d'acide inosinique et d'acide guanosinique par phosphorylation directe du groupe 5'-hydroxy d'inosine et de guanosine, comprenant une exposition d'inosine libre à une étape réactionnelle de phosphorylation et ensuite une exposition d'un sel de métal alcalin de guanosine à une étape réactionnelle de phosphorylation en présence de l'inosine libre, tandis que la transmission de la solution réactionnelle est de l'ordre de 20 à 95 %.
